(19) Europäisches Patentamt
European Patent Office
Office européen des brevets

(11) **EP 4 427 766 A1**

(12) **EUROPÄISCHE PATENTANMELDUNG**

(43) Veröffentlichungstag:
**11.09.2024 Patentblatt 2024/37**

(21) Anmeldenummer: **24161120.1**

(22) Anmeldetag: **04.03.2024**

(51) Internationale Patentklassifikation (IPC):
***A61M 1/16*** (2006.01)  ***A61M 1/36*** (2006.01)

(52) Gemeinsame Patentklassifikation (CPC):
**A61M 1/3656; A61M 1/165; A61M 1/3609;**
**A61M 1/3658;** A61M 2205/18; A61M 2205/3368;
A61M 2205/3553; A61M 2205/52

(84) Benannte Vertragsstaaten:
**AL AT BE BG CH CY CZ DE DK EE ES FI FR GB
GR HR HU IE IS IT LI LT LU LV MC ME MK MT NL
NO PL PT RO RS SE SI SK SM TR**
Benannte Erstreckungsstaaten:
**BA**
Benannte Validierungsstaaten:
**GE KH MA MD TN**

(30) Priorität: **07.03.2023 DE 102023105664**

(71) Anmelder: **B. Braun Avitum AG**
**34212 Melsungen (DE)**

(72) Erfinder:
• **Janik, Waldemar**
**34212 Melsungen (DE)**
• **Krause, Silvie**
**34212 Melsungen (DE)**

(74) Vertreter: **Patentanwälte**
**Ruff, Wilhelm, Beier, Dauster & Partner mbB**
**Kronenstraße 30**
**70174 Stuttgart (DE)**

(54) **VERFAHREN UND SYSTEM ZUM AUTOMATISCHEN CHARAKTERISIEREN EINES GEFÄSSZUGANGS EINES DIALYSEPATIENTEN**

(57) Verfahren zum automatischen Charakterisieren eines Gefäßzugangs (1) eines Dialysepatienten (2), der an eine Dialysemaschine (3) angeschlossenen ist oder angeschlossen war, wobei der Gefäßzugang (1) in Abhängigkeit von einer Rezirkulationsrate charakterisiert wird.

Fig. 1

**Beschreibung**

Bei der Dialyse wird ein Shuntgefäß zur Herstellung eines Gefäßzugangs bzw. Shunts in der Regel mehrmals pro Woche doppelt punktiert, wodurch sich verkalkende Verengungen bzw. Stenosen und Wandaussackungen bzw. Aneurysma bilden und ein überschießendes Wachstum der Gefäßinnenhaut (Intimahyperplasie) provoziert werden können. Diese Veränderungen können im Laufe der Zeit zu einem reduzierten Volumenstrom über den Zugang, d.h. einem reduzierten Shuntfluss, führen.

Der Erfindung liegt die Aufgabe zugrunde, ein Verfahren und ein System zum automatischen Charakterisieren eines Gefäßzugangs eines Dialysepatienten zur Verfügung zu stellen, mittels denen automatisch ein nicht mehr ausreichend funktionaler Gefäßzugang detektierbar ist.

Das erfindungsgemäße Verfahren dient zum automatischen Charakterisieren eines Gefäßzugangs eines Dialysepatienten, der an eine Dialysemaschine angeschlossenen ist oder mindestens einmal angeschlossen war. Der Gefäßzugang weist hierbei herkömmlich einen arteriellen Teil bzw. Zugang und einen venösen Teil bzw. Zugang auf. Der Gefäßzugang wird auch als Shunt bezeichnet. Insoweit sei auch auf die einschlägige Fachliteratur verwiesen.

Erfindungsgemäß wird der Gefäßzugang in Abhängigkeit von einer Rezirkulationsrate charakterisiert. Die Rezirkulationsrate R kann hierbei beispielsweise definiert sein als der Rezirkulationsfluss QR bezogen auf den extrakorporalen Blutfluss QB, d.h. es kann gelten:

$$R[\%] = QR/QB * 100\ \%$$

Die Rezirkulationsrate ist typisch abhängig vom Blutfluss. Da der Blutfluss in der Regel bekannt ist, kann diese Abhängigkeit bei der Charakterisierung berücksichtigt werden, indem beispielsweise die Rezirkulationsrate auf den Blutfluss normiert wird und der Gefäßzugang in Abhängigkeit von der auf den Blutfluss normierten Rezirkulationsrate charakterisiert wird.

Der Gefäßzugang kann beispielsweise quantitativ mit einer Maßzahl charakterisiert werden, wobei höhere Zahlenwerte der Maßzahl beispielsweise einen funktional besseren Gefäßzugang abbilden. Der Gefäßzugang kann weiter beispielsweise qualitativ in unterschiedliche Güteklassen charakterisiert werden, beispielsweise in "sehr gut", "gut", "befriedigend", "ausreichend" und "revisionsbedürftig".

Gemäß einer Ausführungsform wird der Gefäßzugang bei zunehmender Rezirkulationsrate mit einer abnehmenden Qualität charakterisiert.

Gemäß einer Ausführungsform wird die Rezirkulationsrate quantitativ oder qualitativ mittels folgender Schritte bestimmt: Verändern mindestens eines Betriebsparameters der Dialysemaschine, Messen einer Änderung mindestens eines Blutwertes am arteriellen Zugang der Dialysemaschine und Bestimmen der Rezirkulationsrate in Abhängigkeit von der gemessenen Änderung des mindestens einen Blutwerts am arteriellen Zugang der Dialysemaschine.

Gemäß einer Ausführungsform wird die Rezirkulationsrate bei einer zunehmenden gemessenen Änderung mit größeren Werten bestimmt. Je höher die Rezirkulationsrate ist, desto stärker wirkt sich die Änderung des Betriebsparameters auf den bzw. die am arteriellen Zugang gemessenen Blutwerte aus. Die Änderung des oder der gemessenen Blutwerte ist folglich charakteristisch für die Rezirkulationsrate.

Gemäß einer Ausführungsform beeinflusst der mindestens eine Betriebsparameter eine Veränderung eines Dialysatflusses durch den Dialysator der Dialysemaschine.

Die Dialysemaschine kann eine Sensorik am Dialysatausgang aufweisen, beispielsweise einen optischen Sensor oder eine Leitfähigkeitssonde.

Es können zwei aufeinanderfolgende Bypässe zum Ändern des Flusses durch den Dialysator auf einen Wert von nahezu 0 ml/min unterschiedlicher Dauer bewirkt werden, wobei sich ergebende Messsignale der Sensorik ausgewertet werden. Nach einem kurzen Bypass ist das Signal gegebenenfalls rezirkulationsbehaftet. Nach einem langen Bypass ist das Signal rezirkulationsbereinigt. Weichen beide Signale voneinander ab, deutet dies daher auf eine Rezirkulation hin. Das Maß der Abweichung ist abhängig von der Rezirkulationsrate.

Gemäß einer Ausführungsform beeinflusst der mindestens eine Betriebsparameter der Dialysemaschine eine Ultrafiltrationsrate und der mindestens eine Blutwert beinhaltet einen bzw. ist ein Hämatokritwert.

Gemäß einer Ausführungsform wird die Ultrafiltrationsrate erhöht und die Rezirkulationsrate in Abhängigkeit von einer Erhöhung des Hämatokritwerts bestimmt.

Zum Messen des Hämatokritwerts kann die Dialysemaschine beispielsweise einen Hämatokritsensor an ihrem arteriellen Zugang bzw. Schlauchabschnitt aufweisen. Optional kann ein weiterer Hämatokritsensor am venösen Zugang bzw. Schlauchabschnitt vorgesehen sein. Beispielsweise kann die Ultrafiltrationsrate kurzzeitig erhöht werden. Dies bewirkt einen erhöhten Entzug von Wasser und somit einen Anstieg des Hämatokritwerts im venösen Blut. Falls Rezirkulation vorhanden ist, passiert das derart angedickte rezirkulierte Blut den Hämatokritsensor am arteriellen Zugang, wodurch ein erhöhter Hämatokritwert messbar ist. Dies ist für eine qualitative Aussage ausreichend, ob Rezirkulation vorliegt oder nicht. Für eine quantitative Aussage kann zusätzlich am venösen Zugang der Hämatokritwert gemessen werden. Das Verhältnis von venösem zu arteriellem Hämatokritwert bzw. ein Verhältnis von zugehörigen Signalflächen korreliert mit der Höhe der Rezirkulationsrate.

Gemäß einer Ausführungsform beeinflusst der mindestens eine Betriebsparameter eine Veränderung der Temperatur des am venösen Zugang der Dialysemaschine ausströmenden Bluts und der mindestens eine Blutwert beinhaltet bzw. ist eine Temperatur des durch den arteriellen Zugang der Dialysemaschine strömenden Bluts.

Die Dialysemaschine kann beispielsweise eine Temperaturänderungsstelleinheit am venösen Schlauch bzw. Zugang aufweisen. Eine Temperaturänderung kann alternativ auch durch Zugabe eines im Vergleich zum Blut kälteren oder wärmeren Flüssigkeitsvolumens (z.B. NaCl-Lösung) erfolgen. In Flussrichtung des Blutes nachfolgend kann ein optionaler Temperatursensor vorgesehen sein. Weiter ist ein Temperatursensor am arteriellen Schlauch bzw. Zugang vorgesehen.

Die Blut-Temperatur am venösen Zugang kann kurzzeitig verändert werden. Die Temperaturänderung wäre im Falle einer Rezirkulation auch im arteriellen Zugang bzw. Schlauch messbar. Dies ist für eine qualitative Aussage ausreichend, ob Rezirkulation vorliegt oder nicht. Für eine quantitative Aussage kann zusätzlich am venösen Abschnitt bzw. Zugang die Temperatur gemessen werden. Das Verhältnis von venöser zu arterieller Temperatur bzw. das Verhältnis der beiden Flächen unterhalb der Temperaturverläufe korreliert mit der Höhe der Rezirkulationsrate.

Gemäß einer Ausführungsform beeinflusst der mindestens eine Betriebsparameter eine Temperatur einer Dialysierflüssigkeit und der mindestens eine Blutwert beinhaltet bzw. ist eine Temperatur des durch den arteriellen Zugang der Dialysemaschine strömenden Bluts.

Die Dialysemaschine kann ein Heizelement zur Temperierung der Dialysierflüssigkeit aufweisen. Weiter kann ein Temperatursensor am arteriellen Zugang bzw. Schlauch vorgesehen sein, optional zusätzlich am venösen Schlauch bzw. Zugang. Weiter kann ein Temperatursensor optional, d.h. nicht zusätzlich, am Dialysatausgang des Dialysators vorgesehen sein.

Die Temperatur der Dialysierflüssigkeit kann kurzzeitig verändert werden. Diese Temperaturänderung wird im Dialysator an das durchströmende Blut weitergegeben. Die Temperaturänderung wäre im Falle einer Rezirkulation auch im arteriellen Zugang bzw. Schlauch messbar. Dies ist für eine qualitative Aussage ausreichend, ob Rezirkulation vorliegt oder nicht. Für eine quantitative Aussage kann zusätzlich am venösen Abschnitt bzw. Zugang die Temperatur gemessen werden. Das Verhältnis von venöser zu arterieller Temperatur bzw. das Verhältnis der beiden Flächen unterhalb der Temperaturverläufe korreliert mit der Höhe der Rezirkulationsrate.

Zeitverzögert kann der Temperaturbolus innerhalb des Dialysators von der Blutseite wieder auf die Dialysatseite übertreten, sodass am Dialysatausgang ebenfalls eine Temperaturänderung messbar wäre, falls eine Rezirkulation vorliegt.

Gemäß einer Ausführungsform beeinflusst der mindestens eine Betriebsparameter eine Zugabe eines Stoffs, insbesondere von NaCl, in das am venösen Zugang der Dialysemaschine ausströmende Blut und der mindestens eine Blutwert beinhaltet bzw. ist eine Konzentration des Stoffs in dem durch den arteriellen Zugang der Dialysemaschine strömenden Blut.

Der Blutwert kann beispielsweise auch ein arterieller Druck bei einer stehenden Blutpumpe beim Anlegen des Zugangs sein.

Gemäß einer Ausführungsform wird die Konzentration des Stoffs basierend auf einer Schall-Ausbreitungsgeschwindigkeit des durch den arteriellen Zugang der Dialysemaschine strömenden Bluts gemessen.

Die Dialysemaschine kann eine Vorrichtung zur Schallgeschwindigkeitsmessung am arteriellen Zugang bzw. Schlauch und optional am venösen Zugang bzw. Schlauch aufweisen.

Es kann beispielswiese eine NaCl-Lösung in das zum Patienten zurückfließende venöse Blut zugegeben werden. Da sich die Dichten von Blut und NaCl-Lösung unterscheiden, wäre eine Schallgeschwindigkeitsänderung am arteriellen Sensor messbar, falls Rezirkulation vorliegt.

Die Boluszugabe kann manuell oder automatisch erfolgen, falls die Dialysemaschine und das Schlauchsystem über entsprechende Vorrichtungen zur Verabreichung eines Dialysierflüssigkeitsbolus verfügen.

Gemäß einer Ausführungsform wird die Rezirkulationsrate quantitativ oder qualitativ basierend auf einem Vergleich zwischen einer gemessenen Clearance und einer für die Dialysemaschine nominellen Clearance ermittelt.

Die Dialysemaschine kann beispielsweise eine Vorrichtung zur Clearance-Bestimmung aufweisen, beispielsweise in Form eines optischen Sensors am Dialysatausgang oder einer Leitfähigkeitssonde am Dialysierflüssigkeitseingang und einer Sonde am Dialysatausgang. Weitere alternative Sensoren zur Bestimmung der Dialysatzusammensetzung (z.B. Ionenkonzentration) sind denkbar. Weiter kann ein blutseitiger Drucksensor am Bluteingang des Dialysators vorgesehen sein.

Theoretisch erzielbare Performanceparameter eines Dialysators sind bekannt, beispielsweise in einer Tabelle hinterlegt, oder bestimmbar. Dazu gehört insbesondere die Clearance. Die Clearance kann abweichen, wenn sich eine sogenannte Sekundärmembran im Dialysator bildet. Diese Sekundärmembran würde aber auch zu einem Anstieg des Bluteingangsdrucks führen und wäre somit identifizierbar. Die Clearance hängt aber auch von der Rezirkulationsrate ab. Befindet sich der Bluteingangsdruck im Normbereich, d.h. es ist keine Sekundärmembran vorhanden, kann eine verminderte Clearance auf eine Rezirkulation hindeuten.

Clearance kann auf unterschiedliche Art und Weise intradialytisch bestimmt werden.

Beispielsweise kann die Zusammensetzung der Dialysierflüssigkeit kurzzeitig geändert werden und die Leitfähigkeiten am Dialysierflüssigkeitseingang und Dialysatausgang können vor und nach der Änderung gemessen werden.

Weiter kann ein Bypass hergestellt werden, d.h. ein kurzzeitiges Vorbeileiten der Dialysierflüssigkeit am Dialysator, bis zur Einstellung eines diffusiven Gleichgewichts zwischen Blut- und Dialysatseite im Dialysator. Anschließend kann ein Signalextremum am Dialysatausgang gemessen werden. Dieses entspricht dem Bluteingangswert, sodass die Clearance im Anschluss nach an sich bekannten Gleichungen bestimmt werden kann.

Theoretisch erzielbare und praktisch erzielte Clearance-Werte können unter Beachtung des Bluteingangsdrucks miteinander verglichen werden. Weichen die Werte voneinander ab, deutet dies auf Rezirkulation hin.

Clearance kann auch durch kurzzeitige Änderung des Blut- oder Dialysatflusses bestimmt werden, wenn dialysatoreingangs- und ausgangsseitig beispielsweise die Leitfähigkeit gemessen wird.

Jedes der oben beschriebenen Verfahren ermöglicht eine qualitative und/oder quantitative Bestimmung der Rezirkulationsrate. Weiter sind Kombinationen der beschriebenen Varianten denkbar. Eine genaue Bezifferung der Rezirkulationsrate ist nicht zwingend erforderlich. Neben diesen zeitlich punktuellen Werten der Rezirkulationsrate sind auch Trends, d.h. die zeitliche Entwicklung der Werte, zur Charakterisierung des Gefäßzugangs auswertbar. Eine mögliche auswertbare Größe wäre beispielsweise die Steigung einer Regressionsgeraden über einen definierten Zeitraum, der mindestens zwei Messungen umfasst.

Gemäß einer Ausführungsform weist das Verfahren weiter die Schritte auf: Ermitteln der Rezirkulationsrate mittels der Dialysemaschine oder mittels eines anderen Messgeräts, Übertragen der ermittelten Rezirkulationsrate über ein Datennetzwerk an eine zentrale Recheneinheit, Übertragen weiterer Daten, die charakteristisch für die Qualität des Gefäßzugangs sind, über das Datennetzwerk an die zentrale Recheneinheit, und Charakterisieren des Gefäßzugangs mittels der zentralen Recheneinheit basierend auf der Rezirkulationsrate und den weiteren Daten.

Gemäß einer Ausführungsform sind die weiteren Daten ausgewählt aus einer Menge von Daten. Die Menge von Daten enthält mindestens eines der folgenden Daten-Elemente: einen über den Zugang fließenden Volumenstrom, einen Kt/V-Wert, insbesondere gewonnen basierend auf einer Analyse von prä- und/oder postdialytischen Blutproben des Dialysepatienten, dialysepatientenbezogene Daten, insbesondere in Form der Größe, des Alters, des Gewichts und/oder des Geschlechts des Dialysepatienten, ein Datum der Herstellung des Gefäßzugangs, und ein Datum einer vorangegangenen Gefäßzugangsrevision bzw. Shuntrevision.

Gemäß einer Ausführungsform werden zur Charakterisierung des Gefäßzugang die Rezirkulationsrate und die weiteren Daten mittels maschinellen Lernens bzw. sogenannter künstlicher Intelligenz ausgewertet. Anhand von patientenbezogenen Daten können nur Daten zum Lernen verwendet werden, die dem Patienten, für den eine Prognose erstellt werden soll, ähnlich sind. Es kann auswählbar sein, ob die Prognose aus einem Modell aus allen Daten oder aus einem Modell erfolgen soll, das auf einem Ähnlichkeitssubset-Daten-Modell basiert.

Gemäß einer Ausführungsform wird in Abhängigkeit von der Charakterisierung des Gefäßzugangs, insbesondere bei der Charakterisierung des Gefäßzugangs als nicht ausreichend, eine Warnmeldung ausgegeben, beispielsweise in Form eines akustischen Warn-Signals, eines visuellen Warn-Signals, eines haptischen Warn-Signals, usw.

Das erfindungsgemäße System zum automatischen Charakterisieren eines Gefäßzugangs eines Dialysepatienten, der an eine Dialysemaschine angeschlossenen ist oder angeschlossen war, weist auf: eine Dialysemaschine, und eine zentrale Recheneinheit, wobei die Dialysemaschine und die zentrale Recheneinheit jeweils dazu ausgebildet sind, ein oben beschriebenes Verfahren auszuführen.

Die Erfindung wird nachfolgend unter Bezugnahme auf die Zeichnungen detailliert beschrieben. Hierbei zeigen hoch schematisch:

Fig. 1   ein System zum automatischen Charakterisieren eines Gefäßzugangs eines Dialysepatienten in einem Ausgangszustand, und

Fig. 2   einen relevanten Ausschnitt des Systems von Fig. 1 in einem Folgezustand.

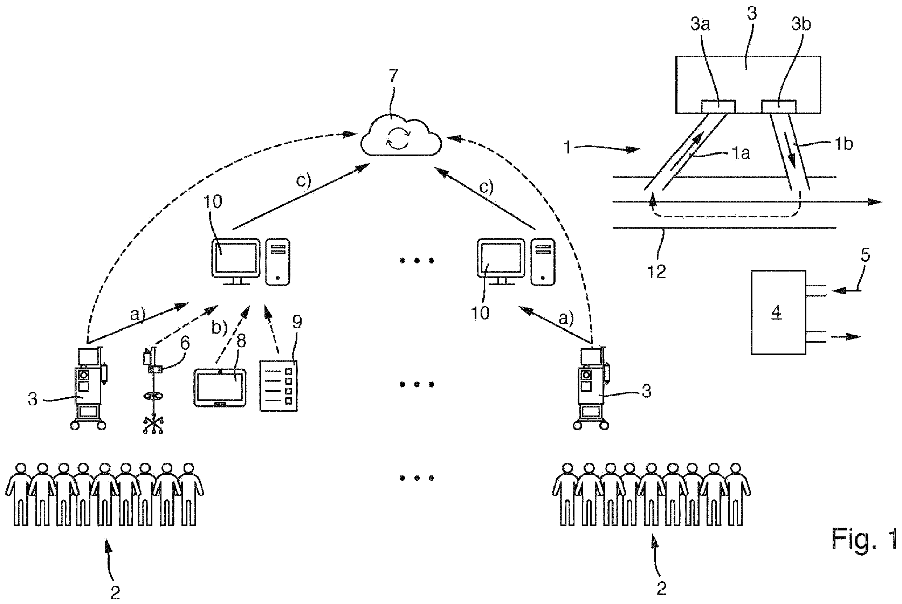

Fig. 1 zeigt hoch schematisch ein System zum automatischen Charakterisieren eines Gefäßzugangs 1 eines Dialysepatienten 2, der an eine Dialysemaschine 3 angeschlossenen ist oder angeschlossen war.

Der Gefäßzugang in ein Gefäß 12 des Patienten 2 weist herkömmlich einen arteriellen Teil 1a und einen venösen Teil 1b auf, wobei der arterielle Teil 1a mit einem korrespondierenden arteriellen Zugang 3a der Dialysemaschine 3 fluidverbunden ist und der venöse Teil 1b mit einem korrespondierenden venösen Zugang 3b der Dialysemaschine 3 fluidverbunden ist.

Das System weist auf: die Dialysemaschine 3 mit einem Dialysator 4, ein optionales Messgerät 6, beispielsweise zur Rezirkulationsratenmessung und Shuntflussmessung, eine zentrale Recheneinheit 7, Komponenten 8 und 9 zur Patientendatenverwaltung und ein Datenmanagementsystem 10.

Die Komponenten 3, 6, 7, 8, 9 und 10 stehen miteinander in Datenverbindung.

Die Komponenten 3, 6, 7, 8 und 9 können als Komponentengruppe, wie dargestellt, mehrfach vorhanden sein.

Die automatische Charakterisierung des Gefäßzugangs 1 des Dialysepatienten 2 läuft wie nachfolgend beschrieben ab.

Zunächst bestimmt die Dialysemaschine 3 die Rezirkulationsrate. Das Ergebnis wird symbolisiert durch das Bezugszeichen a) an das Datenmanagementsystem (DMS) 10 gesendet.

Zusätzlich können weitere Daten, wie eine mittels des Messgeräts 6 messbare Rezirkulationsrate bzw. ein mittels des Messgeräts 6 messbarer Shuntfluss, an das Datenmanagementsystem 10 gesendet werden, symbolisiert durch das Bezugszeichen b).

Weiter können Daten, beispielsweise in Form eines Kt/V-Werts bzw. einer Kt/V-Kurve, an das Datenmanagementsystem 10 gesendet werden, symbolisiert ebenfalls durch das Bezugszeichen b). Der Kt/V-Wert kann beispielsweise auf der Vermessung von prä- und postdialytischen Blutproben in einem Labor basieren.

Schließlich können patientenbezogene Daten, wie z.B. Größe, Alter, Gewicht, Geschlecht, Datum der Gefäßzugangslegung, Datum der letzten Gefäßzugangsrevision, etc., gespeichert in den Komponenten 8 und 9 zur Patientendatenverwaltung, an das Datenmanagementsystem 10 gesendet werden, symbolisiert ebenfalls durch das Bezugszeichen b). Diese Daten können beispielsweise von einem (Tablet-)Computer 8 stammen oder manuell an das Datenmanagementsystem 10 übertragen werden. Weiter können ein Datum der Anlage, eine Art des Zugangs, besondere Ereignisse zu Behandlungen (eintragbar durch den Patienten oder medizinisches Personal, beispielsweise in Form von Nachblutungen), usw. an das Datenmanagementsystem 10 gesendet werden.

Das Datenmanagementsystem 10 überträgt die Daten über ein Datennetzwerk an die zentrale Recheneinheit 7, symbolisiert durch das Bezugszeichen c). Die Recheneinheit 7 befindet sich in einer sogenannten Cloud. Sämtliche Daten werden in der Cloud durch die zentrale Recheneinheit 7 basierend auf maschinellem Lernen verarbeitet.

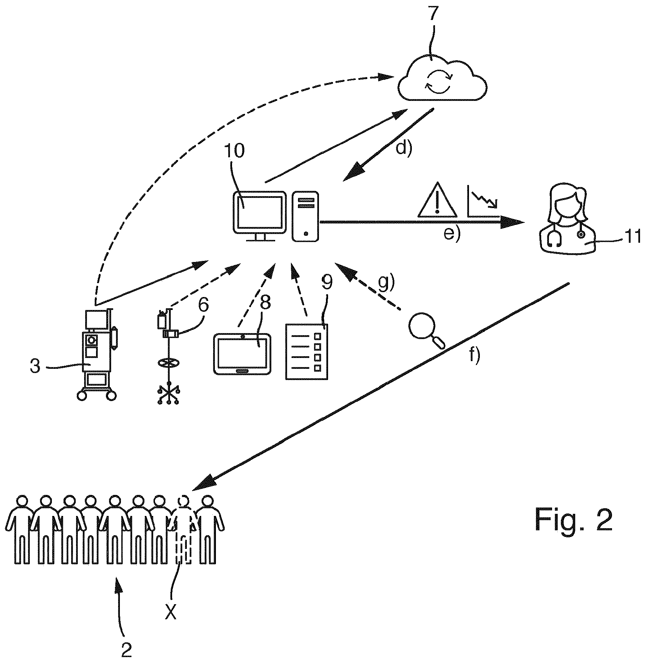

Nun Bezug nehmend auf Fig. 2, meldet die zentrale Recheneinheit 7 nach der Datenanalyse an das Datenmanagementsystem 10, dass sich bei Patient X der Gefäßzugang 1 verschlechtert hat bzw. gerade dabei ist, sich zu verschlechtern, beispielsweise da die ermittelte Rezirkulationsrate im Laufe der letzten Wochen gestiegen ist. Dies ist symbolisiert durch das Bezugszeichen d).

Das Datenmanagementsystem 10 gibt in Folge eine Warnung bzw. einen Hinweis an medizinisches Personal 11 aus, symbolisiert durch das Bezugszeichen e).

Das medizinische Personal 11 untersucht das Gefäß beispielsweise mit einem Stethoskop, einem bildgebenden Verfahren oder einer getriggerten Shuntfluss- oder Rezirkulationsmessung, symbolisiert durch das Bezugszeichen f).

Das Ergebnis dieser Untersuchung kann samt der anderen Daten über das Datenmanagementsystem 10 in die zentrale Recheneinheit 7 gespeist werden, um in der Recheneinheit 7 eine Anpassung des dort ablaufenden Bewertungsalgorithmus durch rekursives Lernen anzustoßen, symbolisiert durch das Bezugszeichen g).

Anders als dargestellt, kann das Datenmanagementsystem 10 Bestandteil der zentralen Recheneinheit 7 sein bzw. das Datenmanagementsystem 10 übernimmt auch die oben genannten Aufgaben der zentralen Recheneinheit 7.

Nachfolgend wird anhand einiger Beispiele beschrieben, wie die Dialysemaschine 3 die Rezirkulationsrate messen kann.

Der Gefäßzugang 1 wird bei zunehmender Rezirkulationsrate grundsätzlich mit einer abnehmenden Qualität charakterisiert.

Zum quantitativen oder qualitativen Bestimmen der Rezirkulationsrate kann mindestens ein Betriebsparameter der Dialysemaschine 3 verändert werden. Anschließend wird eine Änderung mindestens eines Blutwertes am arteriellen Zugang 3a der Dialysemaschine 3 gemessen und die Rezirkulationsrate in Abhängigkeit von der gemessenen Änderung des mindestens einen Blutwerts am arteriellen Zugang 3a der Dialysemaschine 3 ermittelt. Die Rezirkulationsrate wird bei einer zunehmenden gemessenen Änderung mit größeren Werten ermittelt.

Der mindestens eine Betriebsparameter kann eine Veränderung eines Dialysatflusses durch den Dialysator 4 der Dialysemaschine 3 beeinflussen. Alternativ oder zusätzlich kann der mindestens eine Betriebsparameter der Dialysemaschine 3 eine Ultrafiltrationsrate beeinflussen und der mindestens eine Blutwert ist ein Hämatokritwert. Beispielsweise wird die Ultrafiltrationsrate erhöht und die Rezirkulationsrate in Abhängigkeit von einer Erhöhung des Hämatokritwerts bestimmt. Weiter kann der mindestens eine Betriebsparameter eine Veränderung der Temperatur des am venösen Zugang 3b der Dialysemaschine 3 ausströmenden Bluts beeinflussen und der mindestens eine Blutwert ist eine Temperatur des durch den arteriellen Zugang 3a der Dialysemaschine strömenden Bluts. Weiter kann der mindestens eine Betriebsparameter eine Temperatur einer Dialysierflüssigkeit 5 beeinflussen und der mindestens eine Blutwert ist eine Temperatur des durch den arteriellen Zugang 3a der Dialysemaschine 3 strömenden Bluts. Weiter kann der mindestens eine Betriebsparameter eine Zugabe eines Stoffs, insbesondere von NaCl, in das am venösen Zugang 3b der Dialysemaschine 3 ausströmende Blut beeinflussen und der mindestens eine Blutwert ist eine Konzentration des Stoffs in dem durch den arteriellen Zugang 3b der Dialysemaschine 3 strömenden Blut. Die Konzentration des Stoffs kann beispielsweise basierend auf einer Schall-Ausbreitungsgeschwindigkeit des durch den arteriellen Zugang 3b der Dialysemaschine 3 strömenden Bluts gemessen werden.

Weiter kann die Rezirkulationsrate quantitativ oder qualitativ basierend auf einem Vergleich zwischen einer gemessenen Clearance und einer für die Dialysemaschine 3 nominellen Clearance ermittelt werden.

**Patentansprüche**

1. Verfahren zum automatischen Charakterisieren eines Gefäßzugangs (1) eines Dialysepatienten (2), der an eine Dialysemaschine (3) angeschlossen ist oder angeschlossen war, **dadurch gekennzeichnet, dass**

    - der Gefäßzugang (1) in Abhängigkeit von einer Rezirkulationsrate charakterisiert wird.

2. Verfahren nach Anspruch 1, **dadurch gekennzeichnet, dass**

    - der Gefäßzugang (1) bei zunehmender Rezirkulationsrate mit einer abnehmenden Qualität charakterisiert wird.

3. Verfahren nach einem der vorhergehenden Ansprüche, **dadurch gekennzeichnet, dass**

    - die Rezirkulationsrate quantitativ oder qualitativ mittels folgender Schritte bestimmt wird:

        - Verändern mindestens eines Betriebsparameters der Dialysemaschine (3),
        - Messen einer Änderung mindestens eines Blutwertes am arteriellen Zugang (3a) der Dialysemaschine (3) und
        - Bestimmen der Rezirkulationsrate in Abhängigkeit von der gemessenen Änderung des mindestens einen Blutwerts am arteriellen Zugang (3a) der Dialysemaschine (3).

4. Verfahren nach Anspruch 3, **dadurch gekennzeichnet, dass**

    - die Rezirkulationsrate bei einer zunehmenden gemessenen Änderung mit größeren Werten bestimmt wird.

5. Verfahren nach Anspruch 3 oder 4, **dadurch gekennzeichnet, dass**

    - der mindestens eine Betriebsparameter eine Veränderung eines Dialysatflusses durch den Dialysator (4) der Dialysemaschine (3) beeinflusst.

6. Verfahren nach einem der Ansprüche 3 bis 5, **dadurch gekennzeichnet, dass**

    - der mindestens eine Betriebsparameter der Dialysemaschine (3) eine Ultrafiltrationsrate beeinflusst und
    - der mindestens eine Blutwert einen Hämatokritwert beinhaltet.

7. Verfahren nach Anspruch 6, **dadurch gekennzeichnet, dass**

    - die Ultrafiltrationsrate erhöht wird und die Rezirkulationsrate in Abhängigkeit von einer Erhöhung des Hämatokritwerts bestimmt wird.

8. Verfahren nach einem der Ansprüche 3 bis 7, **dadurch gekennzeichnet, dass**

    - der mindestens eine Betriebsparameter eine Veränderung der Temperatur des am venösen Zugang (3b) der Dialysemaschine (3) ausströmenden Bluts beeinflusst und
    - der mindestens eine Blutwert eine Temperatur des durch den arteriellen Zugang (3a) der Dialysemaschine strömenden Bluts beinhaltet; und/oder
    - der mindestens eine Betriebsparameter eine Temperatur einer Dialysierflüssigkeit (5) beeinflusst und
    - der mindestens eine Blutwert eine Temperatur des durch den arteriellen Zugang (3a) der Dialysemaschine (3) strömenden Bluts beinhaltet; und/oder
    - der mindestens eine Betriebsparameter eine Zugabe eines Stoffs, insbesondere von NaCl, in das am venösen Zugang (3b) der Dialysemaschine (3) ausströmende Blut beeinflusst und
    - der mindestens eine Blutwert eine Konzentration des Stoffs in dem durch den arteriellen Zugang (3b) der Dialysemaschine (3) strömenden Blut beinhaltet.

9. Verfahren nach Anspruch 8, **dadurch gekennzeichnet, dass**

    - die Konzentration des Stoffs basierend auf einer Schall-Ausbreitungsgeschwindigkeit des durch den arteriellen Zugang (3b) der Dialysemaschine (3) strömenden Bluts gemessen wird.

10. Verfahren nach einem der vorhergehenden Ansprüche, **dadurch gekennzeichnet, dass**

    - die Rezirkulationsrate quantitativ oder qualitativ basierend auf einem Vergleich zwischen einer gemessenen Clearance und einer für die Dialysemaschine (3) nominellen Clearance ermittelt wird.

11. Verfahren nach einem der vorhergehenden Ansprüche, **gekennzeichnet durch** die Schritte

    - Ermitteln der Rezirkulationsrate mittels der Dialysemaschine (3) oder mittels eines anderen Messgeräts (6),
    - Übertragen der ermittelten Rezirkulationsrate an eine zentrale Recheneinheit (7),
- Übertragen weiterer Daten, die charakteristisch für die Qualität des Gefäßzugangs (1) sind, an die zentrale Recheneinheit (7), und
- Charakterisieren des Gefäßzugangs (1) mittels der zentralen Recheneinheit (7) basierend auf der Rezirkulationsrate und den weiteren Daten.

12. Verfahren nach Anspruch 11, **dadurch gekennzeichnet, dass**

- die weiteren Daten ausgewählt sind aus einer Menge von Daten, die mindestens eines der folgenden Daten-Elemente enthält:
- einen über den Zugang (1) fließenden Volumenstrom,
- einen Kt/V-Wert, insbesondere basierend auf einer Analyse von prä- und/oder postdialytischen Blutproben des Dialysepatienten (2),
- dialysepatientenbezogene Daten, insbesondere in Form der Größe, des Alters, des Gewichts und/oder des Geschlechts des Dialysepatienten (2),
- ein Datum der Herstellung des Gefäßzugangs (1), und
- ein Datum einer vorangegangenen Gefäßzugangsrevision.

13. Verfahren nach Anspruch 11 oder 12, **dadurch gekennzeichnet, dass**

- zur Charakterisierung des Gefäßzugang (1) die Rezirkulationsrate und die weiteren Daten mittels maschinellen Lernens ausgewertet werden.

14. Verfahren nach einem der vorhergehenden Ansprüche, **dadurch gekennzeichnet, dass**

- in Abhängigkeit von der Charakterisierung des Gefäßzugangs eine Warnmeldung ausgegeben wird.

15. System zum automatischen Charakterisieren eines Gefäßzugangs (1) eines Dialysepatienten (2), der an eine Dialysemaschine (3) angeschlossenen ist oder angeschlossen war, aufweisend:

- die Dialysemaschine (3), und
- eine zentrale Recheneinheit (7),
- wobei die Dialysemaschine (3) und die zentrale Recheneinheit (7) dazu ausgebildet sind, ein Verfahren nach einem der vorhergehenden Ansprüche auszuführen.

Fig. 1

EP 4 427 766 A1

Fig. 2

Europäisches Patentamt
European Patent Office
Office européen des brevets

# EUROPÄISCHER RECHERCHENBERICHT

**Nummer der Anmeldung**

EP 24 16 1120

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 2 792 377 A1 (GAMBRO LUNDIA AB [SE]) 22. Oktober 2014 (2014-10-22) * Zusammenfassung; Abbildungen 1,2,7 * * Absätze [0012] - [0024], [0060] - [0061] * | 1-5, 10-13,15 | INV. A61M1/16 A61M1/36 |
| X | US 2019/111200 A1 (MAHESHWARI VAIBHAV [US] ET AL) 18. April 2019 (2019-04-18) * Zusammenfassung; Abbildungen 5-7 * * Absätze [0030], [0065], [0071] - [0076] * | 1-4,6,7, 10-15 | |
| X | DE 197 02 441 C1 (FRESENIUS MEDICAL CARE DE GMBH [DE]) 26. Februar 1998 (1998-02-26) * Zusammenfassung; Abbildungen 1-4 * * Spalte 4, Zeile 18 - Spalte 5, Zeile 51 * | 1,5,8,15 | |
| X | US 5 588 959 A (AHMAD SUHAIL [US] ET AL) 31. Dezember 1996 (1996-12-31) * Zusammenfassung; Abbildungen 1-6 * * Spalte 4, Zeilen 3-48 * | 1,8,15 | **RECHERCHIERTE SACHGEBIETE (IPC)** A61M |
| X | US 6 117 099 A (STEUER ROBERT R [US] ET AL) 12. September 2000 (2000-09-12) * Zusammenfassung; Abbildungen 1-6 * * Spalte 3, Zeilen 32-57 * | 1,8,15 | |
| X | US 2001/050256 A1 (KRIVITSKI NIKOLAI M [US]) 13. Dezember 2001 (2001-12-13) * Zusammenfassung; Abbildungen 1-7 * * Absätze [0006] - [0027] * | 1,8,9,15 | |

Der vorliegende Recherchenbericht wurde für alle Patentansprüche erstellt

| Recherchenort | Abschlußdatum der Recherche | Prüfer |
|---|---|---|
| Den Haag | 12. Juli 2024 | Kaden, Malte |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
........................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 24 16 1120

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

12-07-2024

| Im Recherchenbericht angeführtes Patentdokument | Datum der Veröffentlichung | Mitglied(er) der Patentfamilie | | Datum der Veröffentlichung |
|---|---|---|---|---|
| EP 2792377 A1 | 22-10-2014 | KEINE | | |
| US 2019111200 A1 | 18-04-2019 | AU | 2018352585 A1 | 27-02-2020 |
| | | CA | 3077181 A1 | 25-04-2019 |
| | | CN | 111225695 A | 02-06-2020 |
| | | EP | 3697466 A1 | 26-08-2020 |
| | | US | 2019111200 A1 | 18-04-2019 |
| | | WO | 2019079340 A1 | 25-04-2019 |
| DE 19702441 C1 | 26-02-1998 | DE | 19702441 C1 | 26-02-1998 |
| | | EP | 0900094 A1 | 10-03-1999 |
| | | ES | 2206890 T3 | 16-05-2004 |
| | | JP | 4046770 B2 | 13-02-2008 |
| | | JP | 2001502590 A | 27-02-2001 |
| | | WO | 9832477 A1 | 30-07-1998 |
| US 5588959 A | 31-12-1996 | KEINE | | |
| US 6117099 A | 12-09-2000 | US | 6117099 A | 12-09-2000 |
| | | US | 6582656 B1 | 24-06-2003 |
| US 2001050256 A1 | 13-12-2001 | US | 2001050256 A1 | 13-12-2001 |
| | | US | 2003111423 A1 | 19-06-2003 |
| | | US | 2005051496 A1 | 10-03-2005 |
| | | US | 2005178732 A1 | 18-08-2005 |

EPO FORM P0461

Für nähere Einzelheiten zu diesem Anhang : siehe Amtsblatt des Europäischen Patentamts, Nr.12/82